# EUROPÄISCHE PATENTSCHRIFT

(11) **EP 1 948 030 B1**
(45) Veröffentlichungstag und Bekanntmachung des Hinweises auf die Patenterteilung: **21.07.2010**
(21) Anmeldenummer: 05813640.9
(22) Anmeldetag: 11.11.2005
(51) Int. Cl.: A61B 17/00, A61B 17/12

(54) **OCCLUSIONSINSTRUMENT ZUM VERSCHLIESSEN EINES HERZOHRES**
OCCLUSION INSTRUMENT FOR CLOSING A CARDIAC AURICLE
INSTRUMENT D'OCCLUSION POUR LA FERMETURE D'UNE OREILLETTE CARDIAQUE

(43) Veröffentlichungstag der Anmeldung: 30.07.2008
(73) Patentinhaber: Occlutech GmbH, 07745 Jena (DE)
(72) Erfinder: DAMM, Christoph, 07743 Jena (DE); FIGULLA, Hans-Reiner, 07749 Jena (DE); KLEBON, Susann, 07749 Jena (DE); MOSZNER, Friedrich, 99441 Hohlstedt (DE); MOSZNER, Robert, 07639 Bad-Klosterlausnitz (DE); OTTMA, Rüdiger, 99441 Grossschwabhausen (DE)
(74) Vertreter: Krahbichler, Erik
(86) Internationale Anmeldenummer: PCT/EP2005/012130
(87) Internationale Veröffentlichungsnummer: WO 2007/054116

(56) Entgegenhaltungen:
- DE-B3- 10 338 702
- US-A1- 2003 199 923
- US-A1- 2004 230 222

## Beschreibung

Die vorliegende Erfindung betrifft ein selbstexpandierbares Occlusionsinstrument zum Verschließen eines Herzohres, bestehend aus einem Geflecht dünner Drähte oder Fäden, welches mittels eines Umformungs- und Wärmebehandlungsverfahrens eine geeignete Formgebung erhält, wobei das Occlusionsinstrument einen rückseitigen proximalen Retentionsbereich und einen vorderseitigen distalen Retentionsbereich aufweist, und wobei in dem distalen Retentionsbereich die Enden der Drähte oder Fäden in einer Fassung zusammenlaufen. Des weiteren weist das Occlusionsinstrument einen Mittenbereich zwischen dem proximalen und dem distalen Retentionsbereich auf. Das Occlusionsinstrument ist dabei so ausgeführt, dass es im zusammengefalteten Zustand mittels eines Katheters in den Körper eines Patienten minimal-invasiv einführbar und in dem Herzohr des Patienten positionierbar ist. Des weiteren betrifft die Erfindung ein Verfahren zur Herstellung eines solchen Occlusionsinstruments.

Ein derartiges Occlusionsinstrument ist zumindest teilweise dem Prinzip nach aus der Medizintechnik bekannt. Beispielsweise ist in der DE 10 338 702 vom 22. August 2003 ein Occlusionsinstrument zur Behandlung von Septum- Defekten bekannt, welches aus einem Geflecht dünner Drähte bzw. Fäden besteht und mittels eines Umformungs- und Wärmebehandlungsverfahrens eine geeignete Formgebung erhält. Das bekannte Occlusionsinstrument weist einen proximalen Retentionsbereich, welcher besonders flach ausgeprägt ist, einen distalen Retentionsbereich und einen zylindrischen Steg zwischen dem proximalen und dem distalen Retentionsbereich auf. Am distalen Retentionsbereich laufen die Enden der das Geflecht bildenden Drähte in einer Fassung zusammen. Dabei ist vorgesehen, dass die beiden Retentionsbereiche des bekannten Occlusionsinstruments durch einen meist intravaskulären Operationseingriff beiderseits eines zu verschließenden Shunt in einem Septum zur Anlage kommen, während der Steg durch den Shunt hindurch läuft.

In der Medizintechnik besteht seit längerem das Bemühen, septale Defekte, wie etwa Defekte des Vorhofseptums, mittels eines transvenösen, interventionellen Zugangs nichtchirurgisch, also ohne Operation im eigentlichen Sinne, katheterinterventionell zu verschließen. Dabei wurden verschiedene Occlusionssysteme mit unterschiedlichen Vor- und Nachteilen vorgeschlagen, ohne das sich bisher ein bestimmtes Verschluss-System durchsetzen konnte. Im Folgenden werden die verschiedenen Systeme "Occluder" oder "Occlusionsinstrumente" genannt. Bei allen interventionellen Occlusionssystemen wird transvenös über einen in einem Septum vorliegenden, zu verschließenden Defekt ein selbstexpandierendes Schirmsystem eingebracht. Ein derartiges System könnte beispielsweise aus zwei Schirmchen bestehen, die jeweils an der distalen Seite (d.h. an der weiter von der Körpermitte bzw. vom Herzen entfernten Seite) bzw. an der proximalen Seite (d.h. an der näher zur Körpermitte angeordneten Seite) des Septums positioniert werden, wobei anschließend die beiden Schirmprothesen im Septum-Defekt zu einem Doppelschirm verschraubt werden. Das Verschluss-System besteht somit dann im zusammengebautem Zustand üblicherweise aus zwei aufgespannten Schirmchen, die über einen kurzen, durch den Defekt hindurchlaufenden Stift miteinander verbunden sind.

Bei derartigen aus dem Stand der Technik bekannten Occlusionsinstrumenten stellt es sich jedoch als nachteilig heraus, dass die Implantationsprozedur relativ kompliziert, schwierig und aufwendig ist. Abgesehen von dem komplizierten Implantieren des Verschluss-Systems im zu verschließenden Septum-Defekt besteht bei den verwendeten Schirmchen grundsätzlich die Gefahr der Materialermüdung mit Branchenfraktur. Ferner ist häufig mit thrombembolischen Komplikationen zu rechnen.

Um zu erreichen, dass das erfindungsgemäße Occlusionsinstrument mittels eines Einführbesteckes bzw. eines Führungsdrahtes eingeführt werden kann, ist vorgesehen, dass das Ende des distalen Retentionsbereich eine Fassung aufweist, die in Eingriff mit dem Einführbesteck bzw. Führungsdraht gebracht werden kann. Dabei ist vorgesehen, dass der Eingriff nach der Positionierung des Occlusionsinstruments im Defekt leicht wieder gelöst werden kann. Beispielsweise ist möglich, das Geflecht am Ende des distalen Retentionsbereichs des Occlusionsinstruments derart zu fassen, dass in der Fassung ein Innengewinde hergestellt wird, mit dem das Einführbesteck in Eingriff gelangt. Selbstverständlich sind hier aber auch andere Ausführungsformen denkbar.

Bei einem anderen Occlusionsinstrumenttyp, dem sogenannten Lock-Clamshell-Schirmsystem, sind zwei vorzugsweise mit Dacron bespannte Stahlschirme vorgesehen, die durch je vier Ärmchen stabilisiert werden. Dieser Occludertyp wird über einen venösen Zugang des Patienten implantiert. Bei dem Lock-Clamshell-Occluder hat es sich jedoch als problematisch erwiesen, dass das zur Implantation benötigte Einführbesteck relativ groß ausgeführt werden muss. Ein weiterer Nachteil liegt darin, dass bei anderen Systemen, wie z. B. Amplatzer-Occluder, viele verschiedene Occludergrößen benötigt werden, um den jeweiligen Proportionen des zu schließenden Septum-Defekts gerecht zu werden. So hat sich herausgestellt, dass die Schirmchen im eingesetzten Zustand nicht vollständig abflachen, wenn die Länge oder der Durchmesser des im Defekt eingesetzten Stegs nicht optimal passt. Dies führt zu einer unvollständigen Endothelialisierung. Ferner hat es sich gezeigt, dass viele der im Körper des Patienten implantierten Systeme über einen längeren Zeitraum aufgrund der erheblichen mechanischen Belastung Materialermüdungen und Brüche in den metallischen Strukturen aufweisen. Dies ist insbesondere dann der Fall, wenn zwischen dem Implantat und dem Septum dauerhaft Spannungen bestehen.

Um diese Nachteile auszuräumen, wurden selbst-zentrierende Occlusionsinstrumente entwickelt, die mittels minimalinvasiver Verfahren, beispielsweise über einen Katheter und Führungsdrähte, in den Körper des Patienten eingeführt und in den zu verschließenden Septum-Defekt eingebracht werden. Der Konstruktion liegt dabei das Prinzip zugrunde, dass sich das Occlusionsinstrument auf die Größe des für den intravaskulären Operationseingriff verwendeten Einführbesteckes bzw. Katheter verjüngen lässt. Ein derart verjüngtes Occlusionsinstrument wird dann über den Katheter in den zu verschließenden Septum-Defekt bzw. in den zu verschließenden Shunt des Septum-Defektes eingebracht. Danach tritt der Occluder aus dem Katheter aus, worauf sich anschließend die selbstexpandierenden Schirmchen bzw. Retentionsscheibchen entfalten, die sich beiderseits des Septums anlegen. Die Schirme wiederum enthalten beispielsweise aus Dacron gefertigte Gewebeeinlagen oder werden von solchen überspannt, womit der Defekt bzw. Shunt verschlossen wird. Die im Körper verbleibenden Implantate werden nach einigen Wochen bis Monaten mehr oder weniger vollständig von körpereigenem Gewebe eingeschlossen.

Ein Beispiel eines selbst-zentrierenden Occlusionsinstruments der eingangs genannten Art und gemäß dem Oberbegriff des Patentanspruchs 1 ist aus der WO 99/12478 A1 bekannt, das eine Weiterentwicklung des unter dem Namen "Amplatzer-Occluder" bekannten Occlusionsinstruments gemäß der US-Patentschrift Nr. 5,725,552 ist. Er besteht aus einem Geflecht aus einer Vielzahl feiner, geflochtener Nitinol-Drähte in Form eines Jojos. Jenes Geflecht wird in seiner ursprünglichen Form als Rundgeflecht hergestellt, welches sowohl an seinem Anfang (bzw. an seiner proximalen Seite) als auch an seinem Ende (bzw. an seiner distalen Seite) lose Drahtenden aufweist. Bei der Weiterverarbeitung des Rundgeflechtes müssen dann diese losen Enden jeweils in einer Hülse gefasst und verschweißt werden. Nach dieser entsprechenden Weiterverarbeitung weist sowohl die proximale Seite als auch die distale Seite des fertigen Occluders jeweils eine abstehende Hülse auf. In das distale und proximale Retentionsschirmchen und in dem dazwischen angeordneten Steg sind Dacron-Patches eingenäht. Aufgrund des Memory-Effektes des verwendeten Nitinol-Materials entfalten sich die beiden Retentionsschirmchen beim Verlassen des Katheders selbstständig. Dies erfolgt zunächst über eine ballonartige Zwischenstufe, wobei die Retentions-Schirmchen letztendlich beiderseits des Septums entgültig platziert eine mehr oder weniger abgeplattete Form einnehmen. Der Steg zentriert sich während des Aufspannens der Schirmchen in dem zu verschließenden Shunt selbstständig.

Durch diese beim proximalen Retentionsbereich des Occluders hervorstehende Hülse tritt allerdings das Problem auf, dass das eingesetzte Implantat Embolie bedingte Probleme, insbesondere die konsekutive Embolisation, hervorruft. Diese emboliebedingten Probleme treten insbesondere dann in Erscheinung, wenn der Patient unter einem so genannten Vorhofflimmern des Herzens leidet. Hierbei handelt es sich aus einer frequenten Erregung der Vorhöfe des Herzens, die zu keiner Kontraktion der Vorhöfe führt. Folge dieses Kontraktionsverlustes der Vorkammern des Herzens ist es, dass eine wirksame Durchwirbelung und Durchmischung des Blutes ausbleibt und sich Tromben im Vorhof bilden können. Ein erhebliches Risiko bei der Vorhoftrombenbildung infolge Vorhofflimmerns besteht darin, dass solche Tromben mit dem Blutstrom mitgerissen werden können und in die arterielle Zirkulation gelangen. Folgen dieser Embolisation sind insbesondere Schlaganfälle, die in etwa 5% pro Jahr bei Patienten mit Vorhofflimmern auftreten, falls nicht durch eine chronische Behandlung eine Geringungshemmung des Blutes mit so genannten Dicumerolen durchgeführt wird. Eine Herbeiführung der Geringungshemmung des Blutes mit so genannten Dicumerolen ist allerdings ebenfalls nicht risikolos. Nebenwirkungen der Behandlungen mit Dicumerolen sind vermehrte Blutungen, sodass Konttaindikationen für diese Behandlung bei ca. 20% der Patienten mit Vorhofflimmern besteht und die Patienten somit aufgrund von einer Risikoabwägung Blutung/Schlaganfall das Risiko eines Schlaganfalls in Kauf genommen wird.

Tromben im Vorhof des Herzens entstehen in überwiegender Mehrzahl in den so genannten Herzohren. Die Herzohren sind Ausstülpungen an den Vorhöfen des menschlichen Herzens. Das rechte Herzohr liegt neben der aufsteigenden Aorta, das linke neben der großen Lungenarterie. Dabei ist das linke Herzohr bei Patienten mit Vorhofflimmern der häufige Entstehungsort für Blutgerinnsel, die zu einem Schlaganfall führen können.

Aufgrund der im Zusammenhang mit der zuvor beschriebenen Vorhoftrombenbildung genannten Risiken und Probleme liegt der vorliegenden Erfindung die Aufgabe zugrunde, ein Occlusionsinstrument anzugeben, mit dem das Herzohr des linken Vorhofs verschlossen werden kann, um eine Thrombusbildung mit dem Risiko eines Schlaganfalls erheblich zu reduzieren. Insbesondere soll ein Occlusionsinstrument angegeben werden, mit dem das Schlaganfallrisiko auch bei solchen Patienten reduziert werden kann, bei denen eine Gerinnungshemmung mit Dicumerolen (so genannte Antikoagolation) aufgrund von Blutungsneigungen kontra indiziert ist.

Diese Aufgabe wird mit einem selbstexpandierbaren Occlusionsinstrument zum Verschließen eines Herzohres gemäß Anspruch 1 gelöst.

Die erfindungsgemäße Lösung weist eine Reihe wesentlicher Vorteile gegenüber der aus dem Stand der Technik bekannten und vorstehend genannten Occlusionsinstrumenten auf. Zum einen handelt es sich bei dem erfindungsgemäßen Occluder um ein selbst expandierbates Instrument, was auf besonders einfache Weise mit beispielsweise einem geeigneten Einführkatheter implantierbar ist. Hierzu wird beispielsweise eine Vene im Bereich der Leiste des Patienten punktiert und das Einführkathetersystem bis an die Scheidewand des rechten Vorhofs vorgeführt. Mittels einer Punktion der Scheidewand des Vorhofs, was beispielsweise eine bekannte transseptale Punktion sein kann, wird der linke Vorhof des Herzens erreicht, so dass anschließend von der Leistenvene aus das Einführkathetersystem in das linke Herzohr eingebracht werden kann. Über das Einführkathetersystem kann anschließend das selbstexpandierbare Occlusionsinstrument zum Verschließen des Herzohres eingebracht werden.

Das Occlusionsinstrument, welches während der Implantation im zusammengefalteten Zustand vorliegt, weist in bevorzugter Weise einen Durchmesser von 6 bis 10 frenches auf, so dass der Eingriff zum Verschließen des Herzohres minimal-invasiv ist.

Nachdem das zusammengefaltete Occlusionsinstrument mit Hilfe beispielsweise des Einführkatheters in dem zu verschließenden Herzohr positioniert ist, wird das Occlusionsinstrument von dem Katheter freigegeben, worauf sich dieses infolge seiner selbstexpandierbaren Natur auseinanderfaltet und die mittels des bei Herstellung angewandten Umformungs- und Wärmebehandlungsverfahrens ausgeprägte Formgebung einnimmt. In diesem expandierten Zustand ist der rückseitige proximale Retentionsbereich mit dem daran ausgebildeten Krempenbereich vollständig auseinandergefaltet und kommt an den Innenwandungen des zu verschließenden Herzohres zur Anlage. Dabei dient der proximale Retentionsbereich mit dem daran ausgebildeten Krempenbereich zur Befestigung und Positionierung des expandierten Occlusionsinstruments in dem Herzohr. Der sich von dem proximalen Retentionsbereich in Richtung Herzohröffnung erstreckende Mittenbereich sowie der am distalen Ende des Mittenbereiches vorgesehene distale Retentionsbereich füllen dabei den Öffnungsbereich des Herzohres nahezu vollständig aus, so dass das gesamte expandierte Occlusionsinstrument im eingesetzten Zustand als ein Verschlussstöpsel zum Verschließen des Herzohres dient. Auf diese Weise kann in besonders einfacher und minimal-invasiven Weise eine Thrombusbildung mit dem Risiko eines Schlaganfalls erheblich reduziert werden.

Insbesondere dadurch, dass die Positionierung und Fixierung des Occlusionsinstruments mit Hilfe des an den Innenwandungen des Herzohres zur Anlage kommenden Krempenbereiches gebildet wird, kann bei dem Occlusionsinstrument auf Befestigungshäkchen oder andere Verankerungsmittel verzichtet werden, die üblicherweise bei derartigen Occlusionsinstrumenten zur Fixierung und Positionierung des Instrumentes im Gewebe verwendet werden. Dabei ist insbesondere zu beachten, dass infolge der äußerst dünnwandigen Ausgestaltung des Gewebes in der Umgebung des Herzohres die üblicherweise eingesetzten Befestigungshäkchen keine dauerhafte Befestigung und Positionierung des Occlusionsinstrumentes bereitstellen können. Mit der erfindungsgemäßen Lösung und insbesondere durch den am proximalen Retentionsbereich ausgebildeten Krempenbereich kann die Problematik der Befestigung des Occlusionsinstruments an dem äußerst dünnwandigen und leicht zu verletzenden Herzohrgewebe mittels Häkchen umgangen werden.

Gemäß Anspruch 1 ist für das Occlusionsinstrument vorgesehen, dass der proximale Retentionsbereich mit seinem Krempenbereich derart ausgelegt ist, dass er sich beim Expandieren des Occlusionsinstruments nach außen wölbt, um derart im implantierten Zustand mit den Innenwandungen des Herzohres zur Anlage zu kommen. Mit dieser bevorzugten Ausführungsform ist es demnach möglich, dass das erfindungsgemäße selbstexpandierbare Occlusionsinstrument mittels eines Einführ-Kathetersystems besonders tief in das zu verschließende Herzohr vorgeschoben werden kann. Der distale Retentionsbereich, der in vorteilhafter Weise beispielsweise als distales Schirmchen ausgebildet ist, wird anschließend, das heißt nachdem das Occlusionsinstrument mit Hilfe des Kathetersystems in dem zu verschließenden Herzohr eingeschoben worden ist, zur Entfaltung gebracht und positioniert, wobei das Schirmchen am Rand der Öffnung des Herzohres zum Eingang des Herzohres anliegt. Gleichzeitig expandiert sich auch der proximale Bereich des Occlusionsinstruments, das heißt der proximale Schirm, wobei beim Expansionsvorgang des proximalen Schirmchens der proximale Retentionsbereich des occlusionsinstruments weiter in das Herzohr hineingezogen wird und so über den Mittenbereich eine Zugkraft auf das distale Schirmchen ausgeübt wird. Als eine direkte Folge hiervon wird das distale Schirmchen bzw. der distale Retentionsbereich unter einer permanenten Spannung am Eingang des Herzohres gehalten. Anders ausgedrückt bedeutet dies, dass mit der vorteilhaften Weiterentwicklung des selbstexpandierbaren Occlusionsinstruments ein selbst-positionierendes und selbst-haltendes Occlusionsinstrument angegeben wird, wobei die Position des distalen Schirmchens vorzugsweise bündig an der Öffnung des Herzohres mit Hilfe des sich selbständig nach außen wölbenden proximalen Schirmchens gehalten wird.

Bevorzugte Weiterentwicklungen der Erfindung bezüglich des Occlusionsinstruments sind in den Unteransprüchen angegeben.

Ferner ist für das Occlusionsinstrument in einer bevorzugten Weiterentwicklung vorgesehen, dass der proximale Retentionsbereich eine zum proximalen Ende hin offene Form aufweist. Damit kann in besonders vorteilhafter Weise erreicht werden, dass sich das Occlusionsinstrument - unabhängig von der Proportion des Durchmessers des zu verschließenden Herzohres und unabhängig von der Stärke der Herzohrwand - an der Innenwandung des Herzohres selbstständig anpasst. Damit ist insbesondere der sichere Halt des expandierten Occlusionsinstruments im Herzohr gewährleistet. Durch die Ausbildung des Occlusionsinstruments als ein Gewebe und aufgrund der zum proximalen Ende offenen Form des proximalen Retentionsbereiches kann des weiteren erreicht werden, dass das implantierte Occlusionsinstrument gewissen Eigenbewegungen des Herzohres selbstständig mitmacht, was vor allem im Hinblick auf eine Materialermüdung und eine dauerhafte und zuverlässige Wirkungsweise des Occlusionsinstruments eine wesentliche Rolle spielt. Insbesondere treten die üblichen hiermit im Zusammenhang stehenden Komplikationen nicht mehr auf.

Durch das einerseits flexible und andererseits kraftschlüssige Anliegen des Krempenbereiches an der Innenwandung des Herzohres kann ferner erreicht werden, dass das eingesetzte Occlusionsinstrument deutlich schneller als bei den aus dem Stand der Technik bekannten Verschlusssystemen vollständig von körpereigenem Gewebe eingeschlossen werden kann.

Aus der Verwendung eines aus dünnen Drähten oder Fäden aufgebauten Geflechts als Ausgangsmaterial für das erfindungsgemäße Occlusionsinstrument leitet sich der weitere Vorteil ab, dass es eine langfristige mechanische Stabilität aufweist. Wie bereits angedeutet, kann das Auftreten von Brüchen in der Struktur oder anders artige Materialermüdung des eingesetzten Implantats weitgehend verhindert werden. Ferner besitzt das Geflecht eine ausreichende Steifigkeit. Der am proximalen Ende ausgebildete Krempenbereich zusammen mit die zum proximalen Ende hin offene Formgebung des proximalen Retentionsbereiches des Geflechts gestattet es zusätzlich, dass der proximale Retentionsbereich des Occlusionsinstrument im eingesetzten und expandierten Zustand an den Innenwandungen des Herzohres vollständig abflacht, und zwar nahezu unabhängig von dem Durchmesser der Herzohröffnung oder der Stärke der Innenwandungen des Herzohres. Dadurch, dass am proximalen Retentionsbereich auf eine Fassung zum Zusammenbündeln bzw. Zusammenfassen des Geflechts verzichtet werden kann, ragt auch keine Komponente des Occlusionsinsttuments in das Herzohr weiter hinein, so dass auch keine Abwehrreaktionen des Körpers oder andere möglichen Komplikationen zu befürchten sind.

In einer besonders vorteilhaften Realisierung des erfindungsgemäßen Occlusionsinstruments ist vorgesehen, dass der am proximalen Retentionsbereich gebildete Krempenbereich durch Zurückstülpen des proximalen Retentionsbereiches zum distalen Ende hin ausgebildet ist. Hierbei handelt es sich um eine besonders leicht zu realisierende und dabei wirkungsvolle Weise, den Krempenbereich beim Occlusionsinstrument auszubilden. Insbesondere ist es somit möglich, das gesamte Occlusionsinstrument aus einem einstückigen Geflecht zu bilden, so dass einerseits keine mechanischen Verbindungselemente zwischen dem Krempenbereich und dem proximalen Ende notwendig sind, und andererseits die Dimension des Occlusionsinstruments im zusammengefalteten Zustand weiter minimiert werden kann. Selbstverständlich sind hier aber auch andere Ausführungsformen zum Ausbilden des Krempenbereichs am proximalen Retentionsbereich denkbar.

Um zu erreichen, dass der distale Retentionsbereich des Occlusionsinstruments im implantierten und expandierten Zustand an dem Randsaum der Herzohröffnung vollständig abflacht, und zwar nahezu unabhängig von dem Durchmesser der Herzohröffnung, ist es in einer besonders vorteilhaften Weiterentwicklung der zuvor genannten Ausführungsformen des Occlusionsinstruments vorgesehen, dass der distale Retentionsbereich eine Vertiefung aufweist, in der die Fassung angeordnet ist. Durch das Anordnen der Fassung in der am distalen Ende des Occlusionsinstruments vorgesehenen Vertiefung ragen auch keine Komponenten des Occlusionsinstruments über die Herzohrwandung hinaus, so dass ein ständiger Blutkontakt mit Komponenten des Implantats verhindert werden kann. Dies hat den Vorteil, dass Abwehrreaktionen des Körpers und keine thrombebolischen Komplikationen zu befürchten sind. Insbesondere dadurch, dass sich das Occlusionsinstrument selbstständig in der Öffnung des Herzohrs expandiert, positioniert und fixiert, wobei der distale und proximale Retentionsbereich in radialer Richtung vorgespannt sind, kann das Occlusionsinstrument über einen weiten Bereich unterschiedlich großer Herzohröffnungen eingesetzt werden.

In einer besonders bevorzugten Weiterentwicklung der zuletzt genannten Ausführungsform des erfindungsgemäßen Occlusionsinstruments, bei welcher der distale Retentionsbereich eine Vertiefung aufweist, ist ferner vorgesehen, dass am distalen Ende des Occlusionsinstruments in der Vertiefung ferner ein Verbindungselement angeordnet ist, wobei das Verbindungselement mit einem Katheter in Eingriff bringbar ist. Mit diesem Verbindungselement, welches am Occlusionsinstrument derart angeordnet ist, dass es nicht über die Herzohrwandung hinausragt, wodurch ein ständiger Blutkontakt mit Komponenten des Implantats verhindert werden kann, weist das erfindungsgemäße Occlusionsinstrument ferner die Funktionalität der Rückholbarkeit auf. Andererseits erleichtert ein Verbindungselement, welches mit einem Katheter in Eingriff bringbar ist, das Implantieren und Positionieren des (beim Implantationsvorgang zusammengefalteten) Occlusionsinstrument in dem zu verschließenden Herzohr. Als Verbindungselemente kommen unterschiedliche Einrichtungen in Frage. Denkbar wären beispielsweise Einrastglieder oder auch Haken bzw. Ösen, die mit entsprechend komplementär ausgebildeten Verbindungselementen eines Katheters kraftschlüssig verbunden werden können.

In einer weiteren vorteilhaften Weiterentwicklung ist vorgesehen, dass das Occlusionsinstrument reversibel zusammen- und auseinanderfaltbar ausgeführt ist, so dass das Instrument in seinem expandierten Zustand beispielsweise mit Hilfe eines Explantationskatheters zusammenfaltbar ist, wobei die kraftschlüssige Verbindung zwischen dem am proximalen Retentionsbereich gebildeten Krempenbereich und den Innenwandungen des Herzohres gelöst wird. Dabei ist denkbar, dass zur Explantation ein Katheter beispielsweise an einem am distalen Ende des Occlusionsinstruments gebildeten Verbindungselement eingreift und durch eine externe Manipulation mit Hilfe des Katheters das Zusammenfalten des Occlusionsinstruments bewirkt wird. Somit ist das Occlusionsinstrutrient vollkommen reversibel in den Katheter zurückziehbar, welches das vollständige Entfernen des Instruments ermöglicht.

Um zu erreichen, dass das Geflecht des Occlusionsinstruments mittels eines Umformungs- und Wärmebehandlungsverfahrens seine geeignete Formgebung erhalten kann, ist in einer besonders bevorzugten Ausführungsform vorgesehen, dass das Geflecht auf einem Formgedächtnis-Material, insbesondere Nitinol oder Polymerkunststoff gebildet ist. Der Einsatz von Nitinol bei Occlusionsinstrumenten ist bekannt. Formgedächtnispolymere gehören zur Gruppe der intelligenten Polymere und sind Polymere, die einen Formgedächtniseffekt zeigen, d.h. unter Einwirkung eines äußeren Stimulus, wie z.B. einer Temperaturänderung, ihre äußere Form ändern können.

Dabei wir das Polymer zunächst durch konventionelle Verarbeitungsmethoden, wie etwa Spritzguss oder Extrusion, in seine permanente Form gebracht. Anschließend wird der Kunststoff deformiert und in der gewünschten temporären Form fixiert, was auch "Programmierung" genannt wird. Dieser Vorgang kann bei Polymeren einerseits so erfolgen, dass die Probe erwärmt, deformiert und dann abgekühlt wird. Andererseits kann das Polymer bzw. der Kunststoff auch bei niedriger Temperatur deformiert werden, was als "kaltes Verstrecken" bezeichnet wird. Damit ist die permanente Form gespeichert, während die temporäre Form aktuell vorliegt. Wird nun der Polymerformkörper auf eine Temperatur höher als die Schalttemperatur erwärmt, kommt es zum Auslösen des Formgedächtnis-Effekts und damit zur Wiederherstellung der gespeicherten permanenten Form. Durch Abkühlen der Probe bildet sich die temporäre Form nicht reversibel zurück, weshalb man von einem so genannten Ein-Weg-Formgedächtnis-Effekt spricht.

Im Vergleich zur bekannten Formgedächtnismaterialien wie z.B. der Formgedächtnislegierung Nitinol, einer äquiatomaren Legierung aus Nickel und Titan, sind Formgedächtnispolymere mit ihren Gedächtnisleistungen um ein Vielfaches Überlegen. Dabei ist nur ein geringer Aufwand (Erwärmen bzw. Abkühlen) zur Programmierung der temporären Form bzw. zur Wiederherstellung der permanenten Form nötig. Darüber hinaus beträgt bei Nitinol die maximale Deformation zwischen permanenter und temporärer Form nur 8%. Formgedächtnispolymere weisen wesentlich höhere Verformbarkeit von bis zu 1.100% auf. Sämtliche vorgenannten Formgedächtnispolymere und Materialien werden mit der vorliegenden Erfindung für die biomedizinische Anwendung des erfindungsgemäßen Occlusionsinstruments beansprucht.

In einer vorteilhaften Weiterentwicklung der zuletzt genannten Ausführungsform des erfindungsgemäßen Occlusionsinstruments, bei welchem das Geflecht aus einem Formgedächtnismaterial gebildet wird, ist vorgesehen, dass das Material ein biologisch abbaubares Formgedächtnis-Polymermaterial aufweist. Insbesondere eignen sich synthetische, bioabbaubare Implantatmaterialien. Derartige abbaubare Werkstoffe bzw. Polymere enthalten unter physiologischen Bedingungen spaltbare Bindungen. Dabei spricht man von einer "Bioabbaubarkeit", wenn der Werkstoff unter Verlust der mechanischen Eigenschaft durch oder in einem biologischen System abgebaut wird. Die äußere Form und die Masse des Implantats bleibt während des Abbaus unter Umständen erhalten. Wird von einer Degradationszeit ohne zusätzliche quantifizierende Angaben gesprochen, so ist die Zeit in der der vollständige Verlust der mechanischen Eigenschaft auftritt, gemeint. Unter biostabilen Werkstoffen versteht man solche, die in biologischen Systemen stabil sind und langfristig zumindest nur teilweise abgebaut werden.

Bei abbaubaren Polymeren unterscheidet man zwischen hydrolytisch und enzymatisch abbaubare Polymere. Der hydrolytische Abbau hat den Vorteil, dass die Abbaugeschwindigkeit unabhängig vom Ort der Implantation ist, da Wasser überall vorhanden ist. Demgegenüber ist die Konzentration an Enzymen lokal sehr unterschiedlich. Bei bioabbaubaren Polymeren oder Werkstoffen kann demnach der Abbau durch reine Hydrolyse, enzymatisch induzierte Reaktionen oder durch deren Kombination erfolgen. Typische hydrolysierbare chemische Bindungen sind Amid-, Ester- oder Acetal-Bindungen. Beim Abbau beobachtet man zwei Mechanismen. Beim Oberflächenabbau findet die Hydrolyse chemischer Bindungen ausschließlich an der Oberfläche statt. Aufgrund des hydrophoben Charakters erfolgt der Polymerbau schneller als die Diffusion von Wasser in das Innere des Materials. Diesen Mechanismus beobachtet man vor allem bei Poly(anhydride)n oder Poly(orthoester)n. Für die vor allem für den Formgedächtnis-Effekt bedeutsamen Poly(hydroxcarbonsäuren), wie Poly(milchsäure) oder Poly(glycosesäure) bzw. entsprechende Copolymere, erfolgt der Polymerabbau im gesamten Volumen. Der geschwindigkeitsbestimmende Schritt ist hierbei die hydrolytische Bindungsspaltung, da die Diffusion von Wasser in der eher hydrophylen Polymermatrix relativ schnell erfolgt. Für die Anwendung von bioabbaubaren Polymeren ist entscheidend, dass sie einerseits mit einer Kontrollier- bzw. einstellbaren Geschwindigkeit abbauen und andererseits die Abbauprodukte nicht toxisch sind.

Sämtliche vorgenannten biologisch abbaubaren Formgedächtnis-Polymere werden erfindungsgemäß beansprucht.

Hinsichtlich der Formgebung des erfindungsgemäßen Occlusionsinstruments ist besonders bevorzugt vorgesehen, dass das Occlusionsinstrument eine glockenähnliche Formgebung aufweist, wobei sich das verjüngende Ende der glockenähnlichen Formgebung den distalen Retentionsbereich bildet. Alternativ hierzu kann das Occlusionsinstrument auch eine pilzähnliche Formgebung aufweisen, wobei die Kappe der pilzähnlichen Formgebung den proximalen oder distalen Retentionsbereich bildet. Auch ist denkbar, dass das Occlusionsinstrument eine hantelähnliche Formgebung aufweist, wobei das Mittenstück der hantelähnlichen Formgebung den Mittenbereich zwischen dem proximalen und dem distalen Retentionsbereich des Occlusionsinstruments bildet. Selbstverständlich sind hier aber auch andere Formgebungen denkbar, die je nach Anwendung geeignet auszuwählen sind.

Besonders bevorzugt ist vorgesehen, dass sich das Geflecht des erfindungsgemäßen Occlusionsinstruments auf den Durchmesser eines bei dem minimal-invasiven Operationseingriff verwendeten Katheters verjüngen lässt. Der Vorteil dieser Ausführungsform ist darin zu sehen, dass die zur Implantation und Explantation zu verwendenden Kathetersysteme einen deutlich reduzierten Innendurchmesser aufweisen können, was die Manövrierbarkeit des zu implantierenden Occlusionsinstruments deutlich erhöht. Von daher kann die Positioniergenauigkeit des Instruments im Herzohr verbessert werden. Bei einem aus Nitinol bestehenden Occluder liegt der Innendurchmesser des zur Implantation oder Explantation verwendeten Katheters zwischen 8 bis 10 frenches, wohingegen bei der Verwendung von Occlusionsinstrumenten aus Polymerkunststoff der Innendurchmesser lediglich zwischen 6 bis 8 frenches liegen muss.

Schließlich ist besonders bevorzugt vorgesehen, dass das Occlusionsinstrument wenigstens eine Gewebeeinlage aufweist, die zum vollständigen Verschließen des Herzohrs im bzw. am distalen Retentionsbereich oder im Mittenbereich des Occlusionsinstruments angeordnet ist. Diese Gewebeeinlage dient dazu, die in dem Mittenbereich und in den sich erweiternden Durchmessern des Occlusionsinstruments verbleibenden Zwischenräume nach dem Einsetzen und Expandieren des Instruments im Herzohr zu verschließen. Die Gewebeeinlage wird beispielsweise am distalen Retentionsbereich am Geflecht des Occlusionsinstruments derart befestigt, dass sie wie ein Tuch über den distalen Retentionsbereich gespannt werden kann. Der Vorteil dieser Konstruktion liegt darin, dass sich der Randsaum des distalen Retentionsbereiches bündig an die Herzohröffnung anlegt und weniger Fremdmaterial in den Körper des Patienten eingebracht wird. Die Gewebeeinlagen können beispielsweise aus Dacron hergestellt sein. Selbstverständlich sind hier aber auch andere Materialien und andere Positionen der Gewebeeinlage im bzw. am Occlusionsinstrument denkbar.

Es zeigen:
- Fig.: 1 eine Seitenansicht einer bevorzugten Ausführungsform des erfin- dungsgemäßen Occlusionsinstruments;
- Fig. 2: eine Draufsicht vom proximalen Ende des in Fig. 1 gezeigten Occlu- sionsinstruments;
- Fig. 3: eine perspektivische Ansicht des in Fig. 1 gezeigten Occlusion- sinstruments;
- Fig. 4: das in einem Herzohr des linken Vorhofs eines Patienten implantier- te Occlusionsinstrument gemäß Fig. 1
- Fig. 5: eine stilisierte Vorrichtung zur Fertigung eines Drahtgeflechts, aus welchem das erfindungsgemäße Occlusionsinstrument besteht;
- Fig. 6: das Drahtgeflecht vor der Wärmebehandlung (Tempern); und
- Fig. 7: eine Schnittdarstellung einer mehrteiligen Vorrichtung zur Herstel- lung der Endform eines erfindungsgemäßen Occlusionsinstruments.

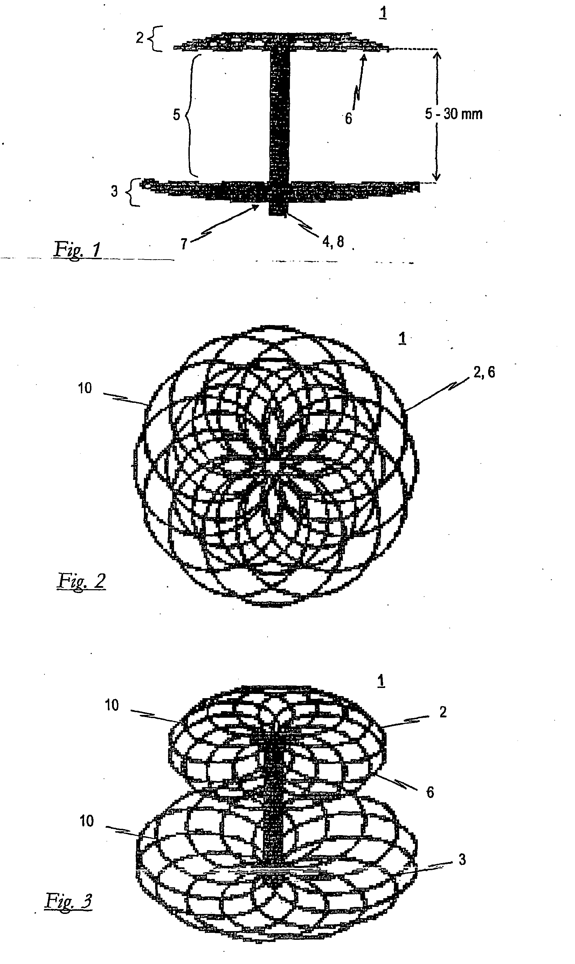

Fig. 1 zeigt eine Seitenansicht einer bevorzugten Ausführungsform des erfindungsgemäßen selbstexpandierbaren Occlusionsinstruments. Figuren 2 und 3 zeigen jeweils eine Draufsicht vom proximalen Ende und eine räumliche Darstellung der Ausführungsform gemäß Fig. 1.

Das erfindungsgemäße Occlusionsinstrument 1 der dargestellten Ausführungsform besteht aus einem Geflecht 10 dünner Drähte oder Fäden, das mit einem Umformungs- und/oder Wärmebehandlungsverfahren eine geeignete Formgebung erhält. Bei den in den Figuren 1 bis 3 dargestellten Formgebungen des Occlusionsinstruments 1 handelt es sich um eine hantelähnliche Formgebung bestehend aus einem vorderseitigen distalen Retentionsbereich 3, einem Mittenbereich 5 und einem rückseitigen proximalen Retentionsbereich 2. In dem distalen Retentionsbereich 3 laufen die Enden der Drähte bzw. Fäden des Geflechts 10 in einer Fassung 4 zusammen. Der proximale Retentionsbereich 2 hingegen weist eine zum proximalen Ende hin offene Form auf.

Des weiteren ist dargestellt, dass der proximale Retentionsbereich 2 einen Krempenbereich 6 aufweist, der durch zumindest teilweises Zurückstülpen des proximalen Retentionsbereiches 2 zum distalen Ende hin gebildet ist.

Das Geflecht 10 ist aus Drähten oder Fäden gebildet, den bevorzugter Weise aus Nitinol oder aus einem anderen Material mit Formgedächtnis oder Memory-Effekt bestehen. Denkbar hierbei wäre es, ein Polymerkunststoff einzusetzen, der die Formgedächtniseigenschaft aufweist. Auch ist es denkbar, ein biologisch abbaubares Formgedächtnismaterial zu verwenden. Wesentlich ist, dass das Geflecht 10 eine ausreichende Flexibilität aufweist, so dass das Occlusionsinstrument 1 auf den Durchmesser eines bei einem minimal-invasiven, insbesondere intravaskulären Operationseingriff verwendeten (nicht explizit dargestellten) Katheters verjüngt werden kann. Aufgrund des Memory-Effekts des Materials verfügt das derart verjüngte Occlusionsinstrument 1 eine Formgedächtnisfunktion, so dass sich das Instrument 1 nach dem Verlassen des Katheters selbstständig expandiert und eine der Verwendung entsprechende, vorbestimmte Form wieder einnimmt. Üblicherweise erfolgt dies, nachdem das zunächst im Katheter angeordnete Occlusionsinstrument 1 an der zu behandelnden Stelle, insbesondere im Herzohr des Herzens eines Patienten, platziert wurde.

Die vorbestimmte Form des Occlusionsinstruments kann auch eine glockenähnliche Form sein, wobei das sich das verjüngende Ende der glockenähnlichen Form den distalen Retentionsbereich 3 bildet. Auch wäre es denkbar, dass das Occlusionsinstrument 1 eine pilzähnliche Formgebung aufweist, wobei die Kappe der pilzähnlichen Formgebung den proximalen oder distalen Retentionsbereich 2, 3 bildet. Die in den Figuren 1 bis 3 dargestellte Ausführungsform des erfindungsgemäßen Occlusionsinstruments weist - wie bereits gesagt - hingegen eher eine hantelähnliche Formgebung auf, wobei das Mittenstück der hantelähnlichen Formgebung den Mittenbereich 5 zwischen dem proximalen und dem distalen Retentionsbereich 2, 3 des Occlusionsinstruments 1 bildet.

Selbstverständlich sind hier aber auch andere, wie auch immer geartete und anwendungsspezifische Formgebungen des Occlusionsinstrumtents denkbar. Die hier dargestellte hantelähnliche Formgebung dient lediglich zur Beschreibung einer bevorzugten Ausführungsform des Occlusionsinstruments und soll insbesondere nicht den Schutzumfang der Erfindung in irgendeiner Weise einschränken.

In den Figuren 1 bis 3 ist das erfindungsgemäße Occlusionsinstrument 1 in seinem expandierten Zustand dargestellt. Wie bereits angedeutet, weist das Occlusionsinstrument 1 einen proximalen Retentionsbereich 2, einen distalen Retentionsbereich 3 sowie einen taillierten, zylindrischen Mittenbereich 5 auf. Der proximale Retentionsbereich 2 mit dem daran ausgebildeten Krempenbereich 6 dient in erster Linie zum Befestigen und Halten des implantierten und expandierten Occlusionsinstruments 1 im Herzohr eines Patienten. Hierzu ist vorgesehen, dass der Krempenbereich 6 eines teilweise in dem zu verschließenden Herzohr an den Innenwandungen des Herzohres zur Anlage kommt und mit den Innenwandungen des Herzohres eine kraftschüssige Verbindung bildet und somit das implantierte und expandierte Occlusionsinstrument 1 in dem Herzohr hält. Denkbar wäre es beispielsweise, dass der proximale Retentionsbereich 2 und/oder der Krempenbereich 6 unter einer radialen Vorspannung stehen, so dass damit für eine relativ breite Variation von Herzohröffnungen der sichere Halt des expandierten Occlusionsinstruments 1 garantiert werden kann.

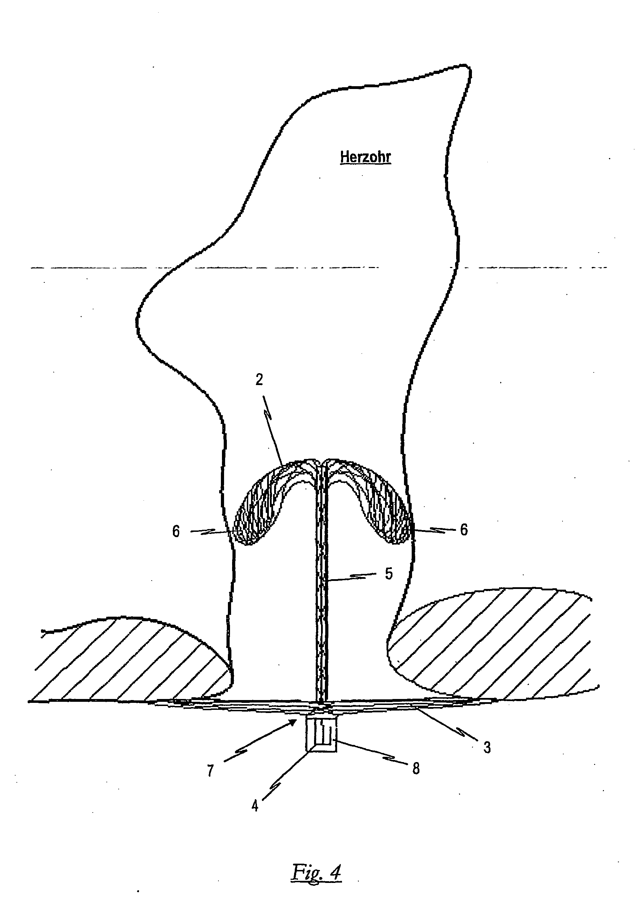

Im implantierten und expandierten Zustand dient der distale Retentionsbereich 3 dazu, die Öffnung des Herzohres möglichst optimal zu verschließen. Auf die Einzelheiten der Funktionsweise der einzelnen Retentionsbereiche wird unter Bezugnahme auf die Fig. 4 im nachfolgenden näher eingegangen.

Der Konstruktion des erfindungsgemäßen Occlusionsinstruments 1 liegt das Prinzip zugrunde, dass sich das Occlusionsinstrument 1 auf die Größe eines Katheters verjüngen lässt. Nach dem Austritt aus dem Katheter entfalten sich dann die Retentionsbereiche 2, 3 selbstständig und legen sich an die Innenwandungen des Herzohres an. Bei der erfindungsgemäßen Konstruktion handelt es sich somit um ein in gewissen Maßen selbstpositionierendes und selbst-zentrierendes System. Der Mittenbereich 5 hat dabei eine für die Anwendung vorab festgelegte Länge, um das Verschließen der Herzohröffnung zu gewährleisten.

Der distale Retentionsbereich 3 weist des weiteren eine Vertiefung 7 auf, in der die Fassung 4 angeordnet ist, in welcher die Enden der Drähte bzw. Fäden des Geflechts 10 zusammenlaufen. Damit kann erreicht werden, dass im implantierten Zustand möglichst keinerlei Material des implantierten Occlusionsinstruments 1 über die Herzohrebene in die Blutbahn des Patienten hineinragen kann. Durch das Vorsehen einer derartigen Vertiefung 7 im distalen Retentionsbereich 3, kann des weiteren ein Verbindungselement 8 am distalen Retentionsbereich 3 angeordnet werden, ohne das eine mögliche Abwehrreaktion vom Körper des Patienten eintritt, da ein Blutkontakt mit dem in der Vertiefung 7 angeordneten Verbindungselement 8 wirksam verhindert wird. Das Verbindungselement 8 kann dahingehend ausgelegt sein, dass es mit einem Katheter in Eingriff bringbar ist.

Durch die flexible Eigenschaft des erfindungsgemäßen Occlusionsinstruments 1 aufgrund der verwendeten Materialien und aufgrund des Geflechtes 10 ist das Instrument 1 reversibel zusammen- und auseinanderfaltbar ausgeführt, so dass ein bereits expandiertes Occlusionsinstrument 1 beispielsweise mit Hilfe eines Explantations- Katheters zusammenfaltbar ist, wobei die kraftschüssige Verbindung zwischen dem Krempenbereich 6 und den Innenwandungen des Herzohres dann gelöst werden.

Fig. 4 zeigt die bevorzugte Ausführungsform des erfindungsgemäßen Occlusionsinstruments 1 im implantierten Zustand. Im einzelnen ist das Occlusionsinstrument im linken Herzohr des Herzen eines Patienten eingesetzt und dient zum Verschließen des Herzohres. Im einzelnen liegt der Krempenbereich 6 des proximalen Retentionsbereiches 2 an den Innenwandungen des Herzohres an und dient zum Positionieren und Fixieren des implantierten Occlusionsinstrumentes 1. Im implantierten Zustand schließt der distale Retentionsbereich 3 mit der Öffnung des Herzohres ab, wobei die Peripherie des distalen Retentionsbereiches an der Wandung der Herzohröffnung zur Anlage kommt, während der Mittenbereich 5 durch die Öffnung hindurch läuft. Das erfindungsgemäße Occlusionsinstrument 1 stellt von daher ein Verschlusssystem dar, dass mittels minimal-invasiver Verfahren, d.h. beispielsweise über einen Katheter und Führungsdrähte, dem Körper eines Patienten zugeführt und an der dafür bestimmten Stelle positioniert wird. Dabei ist insbesondere der distale Retentionsbereich 3 des Instruments 1 derart ausgebildet, dass keinerlei Material des implantierten Occlusionsinstruments 1 über die Herzohrwand in den Blutstrom des Patienten hineinragen kann. Der Rand des distalen Retentionsbereiches 3 schließt dabei bündig mit der Herzohrwand ab. Dies erfolgt über einen relativ weiten Bereich unabhängig von dem Herzohrdurchmesser und der Stärke der Herzohrwandung an der Herzohröffnung. Dadurch kann erreicht werden, dass nach dem Implantieren des Occlusionsinstruments 1 relativ schnell eine vollständige Endothelialisierung eintritt und mögliche Abwehrreaktionen vom Körper des Patienten nicht eintreten, da ein Blutkontakt mit dem Werkstoff des Implantats 1 wirksam verhindert wird.

Obwohl es in den Figuren nicht explizit dargestellt ist, kann das erfindungsgemäße Occlusionsinstrument 1 des weiteren Gewebeeinlagen aufweisen, wobei diese beispielsweise aus dem Material Dacron bestehen können. Denkbar ist dabei die Gewebeeinlagen im Inneren des Mittenbereiches 5 oder am distalen Ende des Retentionsbereiches 3 einzuarbeiten, um die Herzohröffnung vollständig verschließen zu können. Die Einlagerung der Gewebeeinlagen kann z.B. durch Verspannung dieser im Occlusionsinstrument 1 erfolgen. Das im Körper eingesetzte Implantat wird dann bereits nach einigen Wochen bzw. Monaten vollständig von körpereigenem Gewebe eingeschlossen.

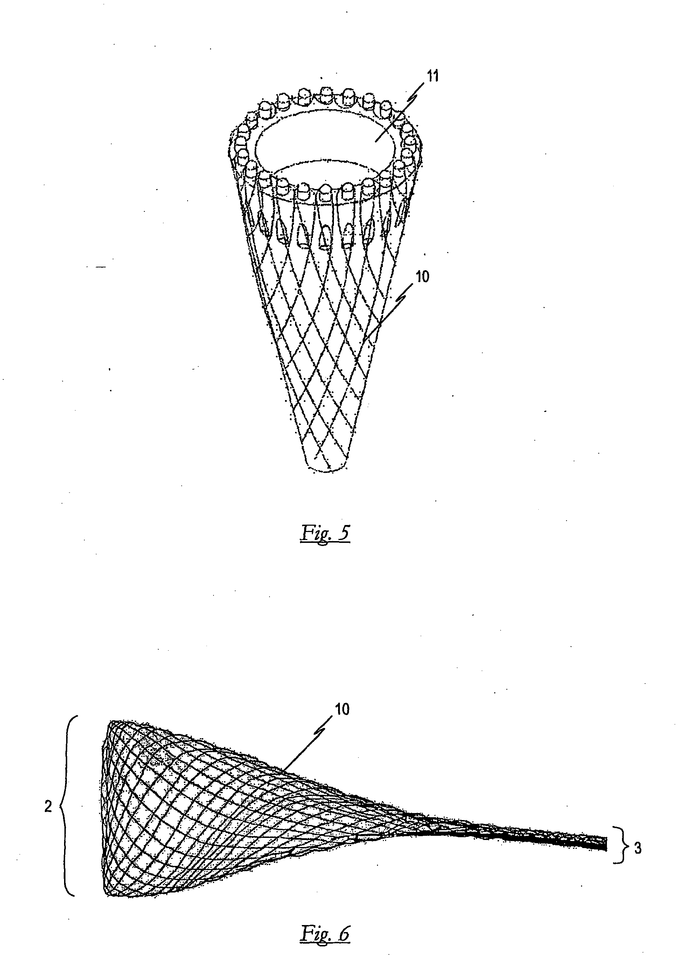

Fig. 5 zeigt eine stilisierte Vorrichtung zur Fertigung eines Drahtgeflechtes. Diese Rundflechtmaschine 11 ist derart ausgeführt, dass es im Betrieb ein trichterförmiges Hohlgeflecht 10 ausbildet, wobei das Hohlgeflecht 10 an einem ersten distalen Ende gebündelt wird und an einem gegenüberliegenden zweiten proximalen Ende offen bleibt.

Fig. 6 zeigt das mit der Rundflechtmaschine 11 ausgebildete trichterförmige Hohlgeflecht 10 in einer separaten Darstellung. Nach dem Ausbilden des trichterförmigen Hohlgeflechts wird der proximale Retentionsbereich 2 am offenen zweiten Ende des Geflechtes 10, der distale Retentionsbereich 3 am gebündelten ersten Ende des Geflechtes 10 und ein zwischen dem proximalen und dem distalen Retentionsbereich 2, 3 angeordneter Mittenbereich mittels einer Wärmebehandlung ausgebildet. Anschließend werden am gebündelten distalen Ende des trichterförmigen Hohlgeflechtes 10 eine Fassung 4 vorgesehen.

Das Ausbilden des Geflechtes 10 für das erfindungsgemäße Occlusionsinstrument ist in der eingangs genannten Patentanmeldung DE 103 38 702 ausführlich beschrieben.

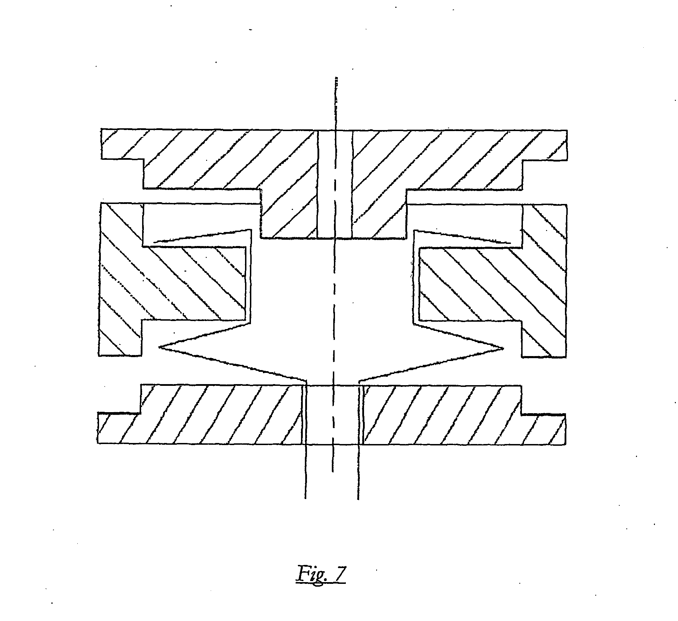

Fig. 7 zeigt eine Schnittdarstellung einer mehrteiligen Vorrichtung zur Herstellung der Endform eines erfindungsgemäßen Occlusionsinstruments.

Es sei darauf hingewiesen, dass die Ausführung der Erfindung nicht auf das in den Figuren beschriebene Ausführungsbeispiel beschränkt ist, sondern auch in einer Vielzahl von Varianten möglich ist.

### Bezugszeichenliste

- 1: Occlusionsinstrument
- 2: proximaler Retentionsbereich
- 3: distaler Retentionsbereich
- 4: Fassung
- 5: Mittenbereich
- 6: Krempenbereich
- 7: Vertiefung
- 8: Verbindungselement
- 10: Geflecht
- 11: Flechtmaschine

## Patentansprüche

1. Selbstexpandierbares Occlusionsinstrument (1) zum Verschließen eines Herzohres, bestehend aus einem Geflecht (10) dünner Drähte oder Fäden, welches mittels eines Umformungs- und Wärmebehandlungsverfahrens eine geeignete Formgebung erhält, mit einem rückseitigen proximalen Retentionsbereich (2) und einem vorderseitigen distalen Retentionsbereich (3), wobei in dem distalen Retentionsbereich (3) die Enden der Drähte oder Fäden in einer Fassung (4) zusammenlaufen, und mit einem Mittenbereich (5) zwischen dem proximalen und dem distalen Retentionsbereich (2, 3), wobei das Occlusionsinstrument (1) im zusammengefalteten Zustand mittels eines Katheters in den Körper eines Patienten minimal-invasiv einführbar und in dem Herzohr des Patienten positionierbar ist,
**dadurch gekennzeichnet, dass**
der proximale Retentionsbereich (2) einen Krempenbereich (6) aufweist, der im expandierten Zustand des Occlusionsinstruments (1) in dem zu verschließenden Herzohr an den Innenwandungen des Herzohres zur Anlage kommt und mit den Innenwandungen des Herzohres eine kraftschlüssige Verbindung bildet und somit das implantierte und expandierte Occlusionsinstrument (1) in dem Herzohr hält, wobei der distale Retentionsbereich (3) die Öffnung des Herzohres verschließt, wobei der proximale Retentionsbereich (2) mit seinem Krempenbereich (6) derart ausgelegt ist, dass er sich beim Expandieren des Occlusionsinstrumentes (1) nach außen wölbt, um derart mit den Innenwandungen des Herzohres zur Anlage zu kommen.

2. Occlusionsinstrument nach Anspruch 1, wobei der proximale Retentionsbereich (2) eine zum proximalen Ende offene Form aufweist.

3. Occlusionsinstrument nach Anspruch 1 oder 2, wobei der Krempenbereich (6) am proximalen Retentionsbereich (2) durch zumindest teilweises Zurückstülpen des proximalen Retentionsbereiches (2) zum distalen Ende gebildet ist.

4. Occlusionsinstrument nach einem der vorhergehenden Ansprüche, wobei der distale Retentionsbereich (3) eine Vertiefung (7) aufweist, in der die Fassung (4) angeordnet ist.

5. Occlusionsinstrument nach Anspruch 4, wobei am distalen Retentionsbereich (3) ferner zumindest ein Verbindungselement (8) in der Vertiefung (7) vorgesehen ist, wobei das Verbindungselement (8) mit einem Katheter in Eingriff bringbar ist.

6. Occlusionsinstrument nach einem der vorhergehenden Ansprüche, wobei das Occlusionsinstrument (1) reversibel zusammen- und auseinanderfaltbar ausgeführt ist, so dass das expandierte Occlusionsinstrument (1) mit Hilfe eines Explantations-Katheters zusammenfaltbar ist, wobei die kraftschlüssige Verbindung zwischen dem Krempenbereich (6) und den Innenwandungen des Herzohres gelöst wird.

7. Occlusionsinstrument nach einem der vorhergehenden Ansprüche, wobei das Geflecht (10) aus einem Formgedächtnismaterial, insbesondere Nitinol oder Polymerkunststoff, gebildet ist.

8. Occlusionsinstrument nach Anspruch 7, wobei das Formgedächtnismaterial ein - biologisch abbaubares Material ist.

9. Occlusionsinstrument nach einem der vorhergehenden Ansprüche, wobei das Occlusionsinstrument (1) eine glockenähnliche Formgebung aufweist, wobei das sich verjüngende Ende der glockenähnlichen Formgebung den distalen Retentionsbereich (3) bildet.

10. Occlusionsinstrument nach einem der Ansprüche 1 bis 8, wobei das Occlusionsinstrument (1) eine pilzähnliche Formgebung aufweist, wobei die Kappe der pilzähnlichen Formgebung den proximalen oder distalen Retentionsbereich (2, 3) bildet.

11. Occlusionsinstrument nach einem der Ansprüche 1 bis 7, wobei das Occlusionsinstrument (1) eine hantelähnliche Formgebung aufweist, wobei das Mittenstück der hantelähnlichen Formgebung den Mittenbereich (5) zwischen dem proximalen und dem distalen Retentionsbereich (2, 3) des Occlusionsinstruments (1) bildet.

12. Occlusionsinstrument nach einem der vorhergehenden Ansprüche, wobei sich das Geflecht (10) auf den Durchmesser eines bei einem minimal-invasiven Operationseingriff verwendeten Katheters verjüngen lässt.

13. Occlusionsinstrument nach einem der vorhergehenden Ansprüche, wobei zumindest eine Gewebecinlage, welche zum vollständigen Verschließen des Herzohrs im bzw. am distalen Retentionsbereich (3) oder im Mittenbereich (5) des Occlusionsinstruments (1) angeordnet ist.

## Claims

1. Self-expandable occlusion instrument (I) for closing an atrial auricle, consisting of a mesh (10) of thin wires or threads given a suitable shape by means of a forming and heat-treatment process, comprising a rear proximal retention area (2) and a front distal retention area (3), the ends of the wires or threads converging in a holder (4) in the distal retention area (3), and comprising a central area (5) between the proximal and distal retention areas (2, 3), wherein the occlusion instrument (1) can be inserted into the body of a patient in the collapsed state and positioned in the atrial auricle of the patient by means of a catheter in a minimally invasive procedure, **characterised in that** the proximal retention area (2) has a flanged area (6) which comes to bear against the inner walls of the atrial auricle when the occlusion instrument (I) is in the expanded state in the atrial auricle to be closed and forms a non-positive connection with the inner walls of the atrial auricle and therefore holds the implanted and expanded occlusion instrument (I) in the atrial auricle, the distal retention area (3) closing the atrial auricle opening, the flanged area (6) of the proximal retention area (2) being designed in such a manner that it distends outwards upon the expansion of the occlusion instrument (1) in order thus to come to bear against the inner walls of the atrial auricle.

2. Occlusion instrument according to claim 1, in which the proximal retention area (2) has a shape open towards the proximal end.

3. Occlusion instrument according to claim 1 or claim 2, in which the flanged area (6) on the proximal retention area (2) is formed by at least partially folding the proximal retention area (2) back towards the distal end.

4. Occlusion instrument according to one of the preceding claims, in which the distal retention area (3) has a recess (7) in which the holder (4) is arranged.

5. Occlusion instrument according to claim 4, in which at least one connecting element (8) is furthermore provided in the recess (7) in the distal retention area (3), wherein the connecting element (8) can be brought into engagement with a catheter.

6. Occlusion instrument according to one of the preceding claims, in which the occlusion instrument (1) is designed in such a manner that it can be reversibly collapsed and expanded so that the expanded occlusion instrument (1) can be collapsed with the aid of an explantation catheter, the non-positive connection between the flanged area (6) and the inner walls of the atrial auricle being released.

7. Occlusion instrument according to one of the preceding claims, in which the mesh (10) is formed of a shape-memory material, in particular nitinol or plastic.

8. Occlusion instrument according to claim 7, in which the shape-memory material is a biodegradable material.

9. Occlusion instrument according to one of the preceding claims, in which the occlusion instrument (1) has a bell-like shape, the tapering end of the bell-like shape forming the distal retention area (3).

10. Occlusion instrument according to one of claims 1 to 8, in which the occlusion instrument (1) has a mushroom-like shape, the cap of the mushroom-like shape forming the proximal or distal retention area (2, 3).

11. Occlusion instrument according to one of claims 1 to 7, in which the occlusion instrument (1) has a dumbbell-like shape, the centre piece of the dumbbell-like shape forming the central area (5) between the proximal and distal retention areas (2, 3) of the occlusion instrument (1).

12. Occlusion instrument according to one of the preceding claims, in which the mesh (10) can be tapered to the diameter of a catheter used in a minimally invasive surgical procedure.

13. Occlusion instrument according to one of the preceding claims, in which at least one fabric insert is arranged in or on the distal retention area (3) or in the central area (5) of the occlusion instrument (1) for complete closure of the atrial auricle.

## Revendications

1. Instrument d'occlusion auto-expansible (1) pour la fermeture d'une oreillette cardiaque, composé d'une tresse (10) en fibres ou fils fins, qui obtient une forme adaptée par procédé de déformation ou de traitement thermique, pourvu d'une zone de rétention proximale (2) sur le côté arrière et d'une zone de rétention distale (3) sur le côté avant, les extrémités des fils ou des fibres aboutissant dans un support dans la zone de rétention (3), et pourvu d'une zone centrale (5) entre la zone de rétention proximale et distale (2, 3), l'instrument d'occlusion (1) pouvant être introduit de manière moins invasive dans le corps du patient à l'état replié au moyen d'un cathéter et pouvant être positionné dans l'oreillette cardiaque du patient, **caractérisé en ce que** la zone de rétention proximale (2) comporte une zone de rebord (6), qui à l'état déplié de l'instrument d'occlusion (1) est disposée dans les parois internes de l'oreillette cardiaque à fermer et forme un assemblage par force avec les parois internes de l'oreillette cardiaque et maintient ainsi l'instrument d'occlusion (1) implanté et déplié dans l'oreillette cardiaque, la zone de rétention (3) distale fermant l'ouverture de l'oreillette cardiaque, la zone de rétention proximale (2) avec sa zone de rebord (6) étant conçue de sorte qu'elle se courbe vers l'extérieur lors du déploiement de l'instrument d'occlusion (1) afin de se positionner ainsi avec les parois internes de l'oreillette cardiaque.

2. Instrument d'occlusion selon la revendication 1, dans lequel la zone de rétention proximale (2) comporte une forme ouverte sur l'extrémité proximale.

3. Instrument d'occlusion selon la revendication 1 ou 2, dans lequel la zone de rebord (6) est conçue sur la zone de rétention proximale (2) par un recouvrement au moins partiel de la zone de rétention proximale vers l'extrémité distale.

4. Instrument d'occlusion selon l'une des revendications précédentes, dans lequel la zone de rétention distale (3) comporte un renfoncement (7), dans lequel le support (4) est disposé.

5. Instrument d'occlusion selon la revendication 4, dans lequel au moins un élément de liaison (8) est par ailleurs prévu dans le renfoncement (7) sur la zone de rétention distale (3), l'élément de liaison (8) pouvant être mis en prise avec un cathéter.

6. Instrument d'occlusion selon l'une des revendications précédentes, dans lequel l'instrument d'occlusion (1) est conçu de manière à pouvoir être déplié et replié de manière réversible de sorte que l'instrument d'occlusion (1) déplié peut être replié à l'aide d'un cathéter d'explantation, l'assemblage par force entre la zone de rebord (6) et les parois internes de l'oreillette cardiaque étant supprimé.

7. Instrument d'occlusion selon l'une des revendications précédentes, dans lequel la tresse (10) est conçue avec un matériau à mémoire de forme, en particulier du nitinol ou un matériau polymère.

8. Instrument d'occlusion selon revendication 7 dans lequel le matériau à mémoire de forme est un matériau biodégradable.

9. Instrument d'occlusion selon l'une des revendications précédentes, dans lequel l'instrument d'occlusion (1) présente une forme de cloche de la zone de rétention distale (3).

10. Instrument d'occlusion selon l'une des revendications 1 à 8, dans lequel l'instrument d'occlusion (1) présente une forme de champignon, le chapeau de la forme de champignon formant la zone de rétention proximale ou distale (2, 3).

11. Instrument d'occlusion selon l'une des revendications 1 à 7, dans lequel l'instrument d'occlusion (1) présente une forme d'haltère, la pièce centrale de la forme d'haltère formant la zone centrale (5) entre la zone de rétention proximale et distale (2, 3) de l'instrument d'occlusion.

12. Instrument d'occlusion selon l'une des revendications précédentes, dans lequel la tresse (10) peut être rétrécie au diamètre d'un cathéter utilisé pour une opération moins invasive.

13. Instrument d'occlusion selon l'une des revendications précédentes, dans lequel au moins un ajout de tissu est disposé pour la fermeture totale de l'oreillette cardiaque dans et sur la zone de rétention distale (3) ou dans la zone centrale (5) de l'instrument d'occlusion.
